# DEMANDE DE BREVET EUROPEEN

(11) **EP 0 903 153 A1**
(43) Date de publication de la demande: **24.03.1999**
(21) Numéro de dépôt: 98402275.6
(22) Date de dépôt: 16.09.1998
(51) Int. Cl.: A61L 2/26

(54) **Procédé et conteneur pour la stérilisation du linge**

(30) Priorité: 19.09.1997 FR 9711677
(71) Demandeur: Regie Linge Developpement, 44300 Nantes (FR)
(72) Inventeur: Patois, Denis Noel, 44470 Carquefou (FR)
(74) Mandataire: Laget, Jean-Loup

(57) **Abrégé**

Un procédé de traitement de linge chirurgical soumis à stérilisation, dans lequel le paquet de linge à traiter est trié, décontaminé, lavé et séché avant d'être conditionné pour le passage en autoclave. Avant stérilisation, ledit paquet de linge est roulé en cylindre et logé dans un tube (2) comportant à au moins une de ses deux extrémités un système de fermeture (4), protégé contre les manipulations, muni d'un filtre, ledit tube étant équipé à sa périphérie de moyens de gerbage (3).

## Description

La présente invention concerne la blanchisserie industrielle et plus spécialement le traitement des linges utilisés en chirurgie qui doivent répondre à des critères très élevés d'asepsie, de stérilisation, et de maintien de la chaîne stérile entre l'usine de traitement et le bloc opératoire.

Les grands établissements hospitaliers et les cliniques chirurgicales suppriment progressivement leurs services internes de blanchisserie pour sous-traiter ce travail à des entreprises spécialisées, équipées pour garantir la parfaite asepsie des linges.

Cependant, il subsiste encore chez les utilisateurs, particulièrement les pharmaciens hospitaliers, une certaine défiance vis-à-vis de la sous-traitance industrielle de la blanchisserie hospitalière, principalement en raison de ce que le risque de rupture de la chaîne de l'asepsie n'est pas totalement éliminé, tout particulièrement en phase finale du traitement, et plus précisément lors de la phase de conditionnement du linge stérilisé.

Les paquets de linges chirurgicaux (champs opératoires, et tenues des praticiens) sont constitués en unités normalisées spécifiques par type d'opération (paquets), pliées puis insérées dans une enveloppe de stérilisation en tissu, puis emballées sous vide après stérilisation et mises en conteneurs pour le transport jusqu'à leur lieu d'utilisation.

Alternativement, les paquets sont placés dans des boîtes de stérilisation mises en conteneurs après stérilisation.

Dans le premier cas, la nécessité de manipuler les enveloppes en tissu, même en chambre stérile, pour les emballer sous vide dans un film transparent, induit, malgré les précautions prises, un risque de recontamination des linges après stérilisation, en raison du contact manuel inévitable entre les opérateurs et les enveloppes en tissu, dont l'emballage sera ouvert en salle d'opération.

Un premier but de l'invention est donc de trouver un moyen de modifier la phase finale du processus de stérilisation pour supprimer ce dernier risque de rupture de la chaîne de l'asepsie.

Dans le second cas, le coût des boîtes de stérilisation actuellement sur le marché et leur relative complexité mécanique (voir par exemple les brevets WO 9004981 et WO 9007346) empêchent une adoption industrielle de cette technique en raison des investissements qu'elle implique pour une entreprise devant servir simultanément un nombre important d'établissements de chirurgie avec un stock de sécurité convenable (environ 10 jours d'activité de chaque établissement).

C'est pourquoi le conditionnement des linges de bloc opératoire en boîtes de stérilisation est jusqu'à présent adopté quasi-exclusivement par les établissements qui possèdent leur propre service de blanchisserie.

Un second but de l'invention est donc de trouver un moyen qui supprime tout risque de rupture de la chaîne de l'asepsie pour un coût comparable ou légèrement supérieur au procédé de stérilisation en enveloppes de tissu et très largement inférieur à celui des boîtes de stérilisation (dans un rapport d'environ 1: 10)

Selon l'invention, le paquet de linge décontaminé, lavé et séché est, avant la stérilisation, roulé en cylindre et logé dans un tube comportant à au moins une de ses extrémités un système de fermeture, protégé contre les manipulations, muni d'un filtre, ledit tube étant équipé à sa périphérie de moyens de gerbage.

L'emploi d'un tube muni de moyens de gerbage comporte de nombreux avantages, en particulier un coût de fabrication extrêmement faible par rapport aux boîtes parallélepipédiques, une résistance naturelle plus importante aux chocs, déformations et agressions, et une amélioration de la circulation des flux dans l'autoclave ce qui rend plus efficace la stérilisation à coeur, efficacité à laquelle contribue également la disposition roulée du linge et la pénétration de vapeur par l'une ou les deux extrémités du tube.

De même, le procédé selon l'invention permet de supprimer deux étapes du procédé de stérilisation en enveloppes de tissu à savoir :
La prise manuelle des enveloppes des paquets stérilisés (point de rupture de la chaîne de l'asepsie) et leur ensachage sous vide.

L'invention concerne donc à la fois le procédé de traitement du linge et le tube spécialement destiné à sa mise en oeuvre.

D'autres caractéristiques et avantages de l'invention ressortiront de la description ci-après du procédé de traitement et d'exemples de réalisation du tube en référence aux dessins annexés sur lesquels :

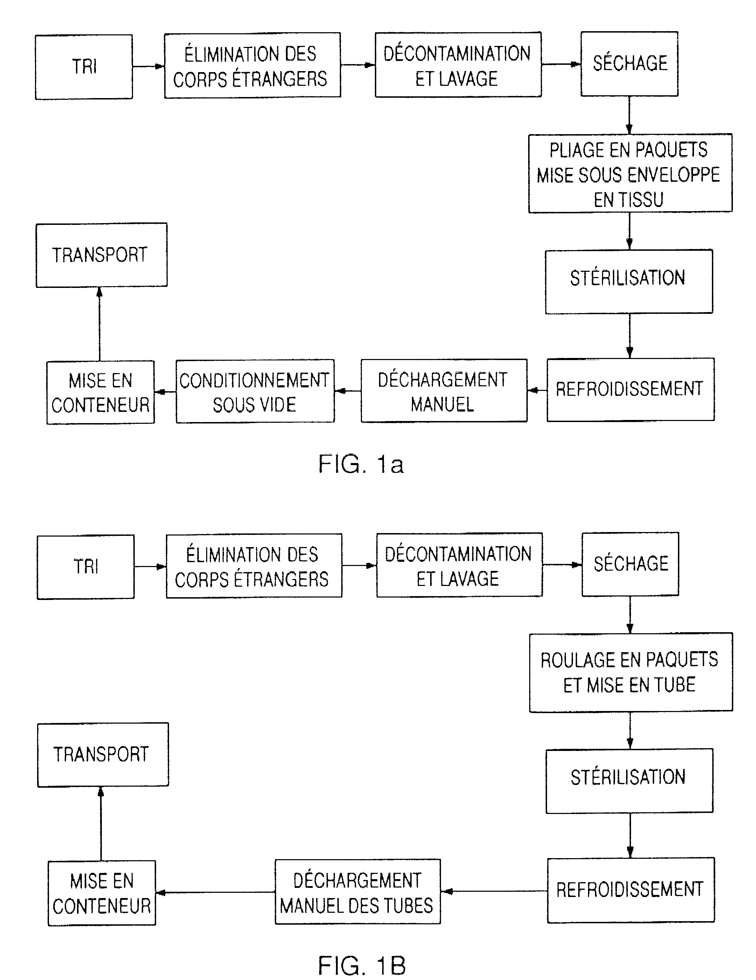

La figure 1a est un diagramme du processus de traitement actuel de linge hospitalier stérilisé et conditionné plié dans une enveloppe de tissu protégé par un emballage réalisé sous vide.
La figure 1b est un diagramme du processus selon l'invention avec utilisation d'un tube spécialement conçu à cet effet ;
La figure 2a est une vue générale en perspective du tube selon l'invention en condition fermée ;
La figure 2b est une vue identique à la vue 2a, l'un des systèmes de fermeture du tube étant ouvert ;
La figure 3 est une vue en coupe selon l'axe vertical médian d'une portion de tube selon les figures 2a et 2b avec un système de fermeture fermé à l'une des extrémités du tube ;
La figure 4a est une vue en perspective de l'avant du couvercle de fermeture du tube ;
La figure 4b est une vue en perspective de l'arrière dudit couvercle ;
La figure 5a est une vue en perspective de l'avant du sur-couvercle venant se combiner avec le couvercle des figures 4a et 4b ;
La figure 5b est une vue en perspective de l'amère dudit sur-couvercle ;
La figure 6 est une vue en perspective de dessus du support de filtre logé dans le couvercle des figures 4a et 4b ;
La figure 7a est une vue en perspective de l'avant du carré de gerbage fixé au tube ;
La figure 7b est une vue en perspective de l'arrière dudit carré de gerbage ;
La figure 8a est une vue générale en perspective de dessus du fermoir à grenouillère utilisé en combinaison avec le carré de gerbage des figures 7a et 7b ;
La figure 8b est une vue générale en perspective de la poignée s'articulant sur ledit carré de gerbage.

Typiquement, le cycle de traitement de linge opératoire conditionné en paquets stériles spécialisés dans une enveloppe de tissu est le suivant :
1. tri article par article, puis par lot et par client,
2. suppression des corps étrangers (pinces, compresses, etc.)
3. lavage et décontamination,
4. séchage,
5. pliage manuel, constitution des packs opérateurs et mise en place du témoin de stérilisation et de l'étiquette de contrôle, mise sous enveloppe,
6. stérilisation,
7. refroidissement,
8. déchargement manuel des lots stérilisés,
9. conditionnement sous vide,
10. mise en conteneurs,
11. transport isotherme jusqu'au site client.

Selon le procédé conforme à l'invention, la cinquième étape du procédé est modifiée en ce que le linge n'est plus plié mais roulé et n'est plus mis sous enveloppe en tissu, mais dans un tube muni de moyens de gerbage et, à au moins une de ses deux extrémités, d'un système de fermeture protégé contre les manipulations, et portant des moyens de filtration ; est également modifiée l'étape de déchargement (huitième étape) puisque l'opération, tout en restant manuelle, supprime tout risque de contact humain, même indirect, avec le linge, enfin la neuvième étape, conditionnement sous vide dans un emballage transparent, est purement et simplement supprimée. Cependant, pour que le processus conforme à l'invention soit efficace, il doit respecter deux contraintes essentielles ;
- la température et la pression à l'intérieur de l'autoclave doivent être exactement reproduites à l'intérieur de chaque tube,
- en sortie d'autoclave, le tube doit constituer une barrière microbienne parfaitement étanche jusqu'à son ouverture dans l'antichambre stérile de la salle d'opération, par du personnel agréé.

La première contrainte est respectée avec un tube contenant le linge roulé et fermé par des bouchons avec respect d'une limite d'asepsie entre le cylindre de linge et les extrémités du tube, la deuxième par l'agencement particulier du système de fermeture du tube et l'adoption d'un disque filtrant qui assure à la fois une bonne pénétration de la vapeur au coeur du linge et, une bonne extraction de l'eau après stérilisation (séchage) ainsi qu'une fonction efficace de barrière antimicrobienne en cours d'utilisation.

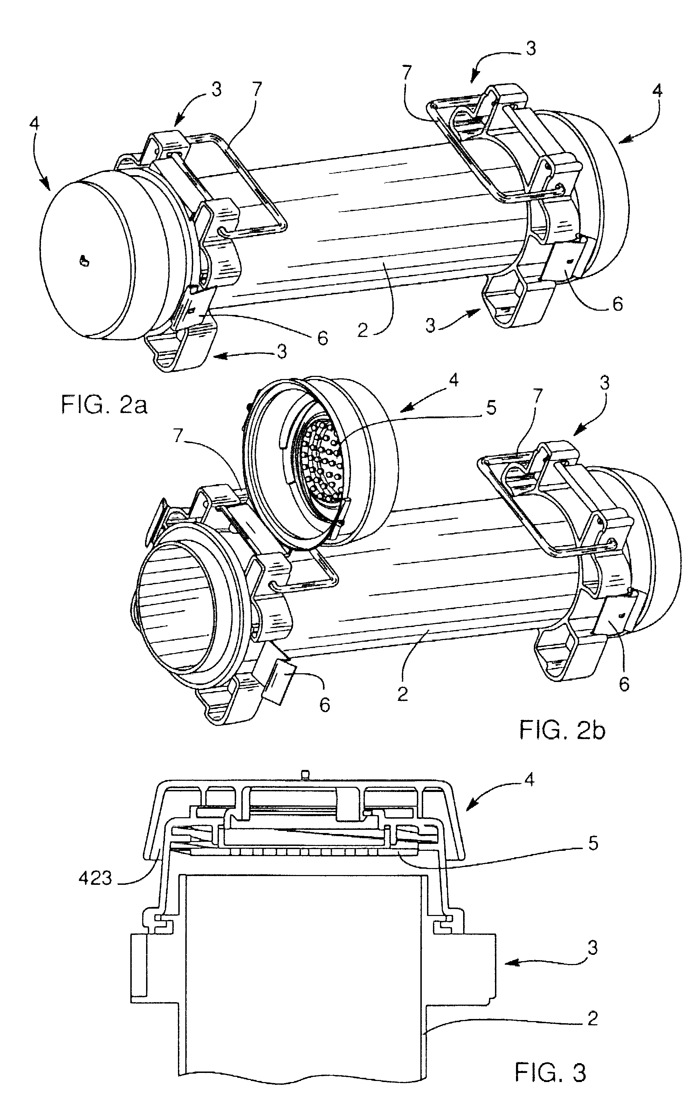

Le tube de stérilisation représenté sur les figures 2a et 2b est constitué extérieurement des pièces principales suivantes : un corps tubulaire lisse 2, deux carrés de gerbage 3 portant les moyens de fermeture (couvercle et sur-couvercle) 4, un support de filtre 5, porté par chaque couvercle, des moyens d'attache rapide 6 portés par le carré gerbage 3 pour coopérer avec chaque couvercle et des moyens de poignée 7 portés également par les carrés de gerbage 3.

Le tube 2 est en tous matériaux appropriés capables de résister à plusieurs cycles de stérilisation sans être déformés ; il doit être inerte par rapport aux produits à stériliser, résister à la corrosion et être le moins sensible possible aux phénomènes de condensation.

A titre d'exemple préférentiel, et sans que cela constitue une limitation de l'invention, le tube sera fabriqué dans une matière synthétique ; de préférence on choisira un polysulfone (PSU) ou un polyméthylpentène (PMP) qui présentent toutes les caractéristiques requises : ils sont utilisables pour des applications médicales, stérilisables, résistent aux températures élevées (environ 135° C), ne gênèrent aucune contrainte dynamique et de faibles contraintes statiques, ont une résistance aux pH élevés, résistent aux chocs, sont de qualité alimentaire et présentent une grande stabilité dimensionnelle.

On a déterminé que la longueur du tube doit de préférence être supérieure à la dimension du paquet de linge roulé à l'intérieur de manière à obtenir une garde qui évite toute possibilité de contact direct à l'ouverture avec des parties de l'appareil (poignées, couvercles, etc...) qui ont pu se trouver soumises à une pollution microbienne notamment pendant la manutention en sortie de stérilisation, et le transport entre l'usine de traitement et le sas de décontamination qui commande l'accès à la salle d'opération (limite d'asepsie).

Avantageusement, on utilisera un tube d'une longueur d'environ 500 mm pour un diamètre extérieur d'au moins 130 mm.

La longueur de 500 mm est choisie pour que l'appareil, muni de ses accessoires (carrés de gerbage, systèmes de fermeture), reste dans un gabarit maximal de 600 mm x 200 mm, ce qui permet d'optimiser le remplissage des autoclaves les plus courants du marché.

Le diamètre extérieur de 130 mm, en combinaison avec la forme roulée du paquet de linge assure d'une part une élimination des poches d'air à l'intérieur du linge ce qui favorise une meilleure pénétration à coeur de la vapeur de stérilisation, et d'autre part une géométrie qui s'insère parfaitement dans le flux de vapeur des autoclaves du marché.

La forme en tube de l'appareil permet en outre d'obtenir une diminution des coûts de fabrication, une très grande facilité d'insertion et d'extraction du linge, une très grande facilité de nettoyage. Elle permet de surcroît d'adapter la longueur de l'appareil aux dimensions des paquets de linge par simple tronçonnage du tube, donc de proposer une gamme adaptée sans faire d'investissements supplémentaires par rapport à ceux que nécessitent la fabrication du modèle de base.

De préférence, également, le tube sera fabriqué dans un matériau transparent pour permettre une vision directe des paquets à travers le tube et surtout une lecture directe de l'étiquette de contrôle placée à la périphérie du paquet avant sa mise en tube.

Le tube est équipé, près de chacune de ses deux extrémités, d'un carré de gerbage 3 qui a pour fonction essentielle de permettre un gerbage des tubes à l'intérieur de l'autoclave et dans les conteneurs de transport avec un espacement suffisant pour permettre une bonne circulation du flux de vapeur entre les tubes lors de la stérilisation, tout en permettant un remplissage optimal des autoclaves et des conteneurs de transport.

Le carré de gerbage, qui définit en section l'encombrement hors tout de l'appareil, peut être obtenu par tout moyen approprié tel que des plots, venus de moulage ou rapportés, des cloisons, etc...

Dans l'exemple de réalisation représenté, en vue de réduire le nombre de pièces nécessaires, et d'optimiser l'ergonomie de l'appareil, les carrés de gerbage sont utilisés simultanément comme supports des systèmes de fermeture et des moyens de manutention.

On voit sur les figures 2a, 2b, 3, 7a et 7b que les carrés de gerbage 3 sont constitués comme des colliers 31, adaptés à la périphérie externe du tube, portant à leur périphérie quatre ensembles 32, venus de moulage, disposés à 90° les uns des autres, lesdits ensembles 32 formant les pieds de gerbage 321 et les supports d'articulation 322 des moyens de fermeture 4 et des attaches 6. Les colliers de gerbage 3 sont fixés à proximité des ouvertures du tube 2 par tout moyen qui évite un perçage ou un affaiblissement dans l'épaisseur du tube, par exemple par collage ou soudage. Ils participent également au renforcement du tube et en particulier reculent la limite de fléchissement de ce dernier.

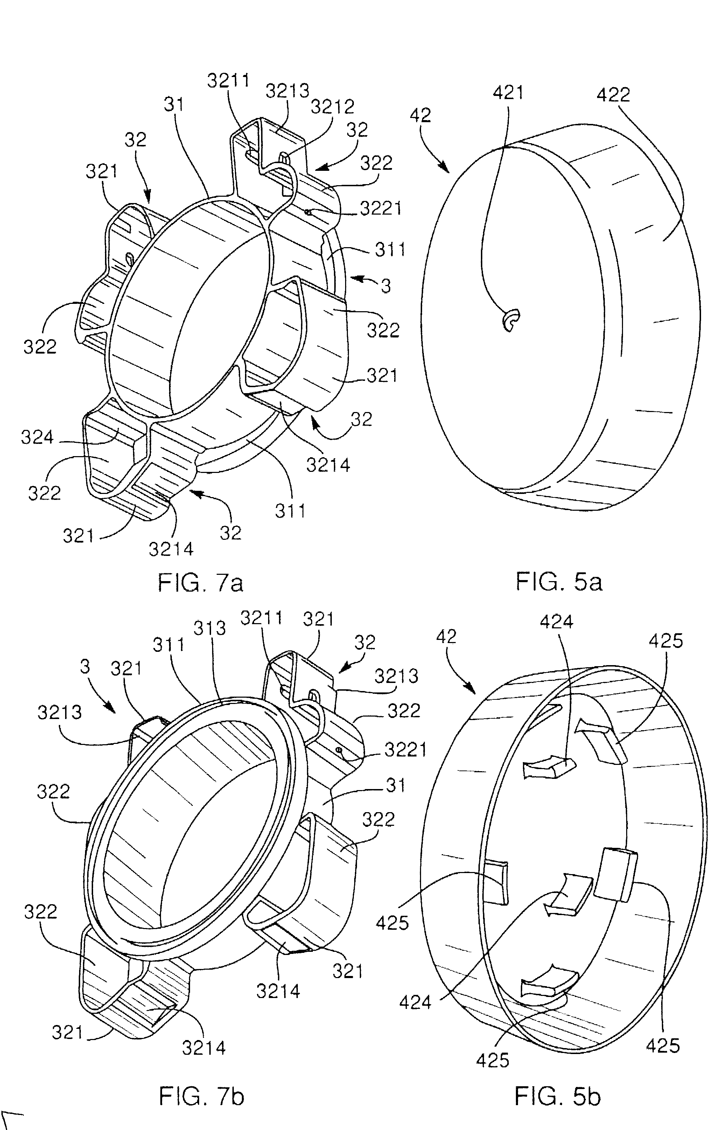

Comme on le voit mieux sur les figures 7a et 7b, les colliers 31 comportent, sur leur face tournée vers l'ouverture adjacente du tube 2, une nervure périphérique 311 dont le chant sert d'appui pour les moyens de fermeture et une gorge 313 pour un point d'étanchéité, de préférence en silicone, non représenté. Les ensembles 32 sont disposés de manière que les pieds de gerbage 321 définissent un plan horizontal inférieur et un plan horizontal supérieur, tandis que les supports d'articulation 322 des moyens d'attache 5 définissent deux plans latéraux verticaux.

Les faces en regard des deux pieds de gerbage 321 de même plan horizontal comportent deux lumières oblongues 3211 horizontales destinées à recevoir les bouts de l'axe d'articulation d'un moyen de ferrneture 4, tandis que les faces opposées desdits pieds de gerbage 321 comportent des trous 3212 destinés à recevoir les extrémités des moyens de poignée 7.

On note sur la figure 7a et 7b que les pieds de gerbage 321 supérieurs comportent un gradin 3213 dont la forme concorde avec un évidement 3214 des pieds de gerbage inférieurs, pour former un moyen de calage des appareils lors du gerbage.

On voit également que les faces en regard des supports d'articulation 322 comportent des trous 3221 destinés à recevoir les bouts de l'axe d'articulation du moyen d'attache 6.

Un exemple particulièrement simple de moyen de poignée 7 est représenté sur la figure 8b, sous la forme d'une simple tige pliée définissant un parallélépipède rectangle à coins arrondis, ouvert sur l'un de ses grands côtés et dont les extrémités libres 71 sont destinées à être insérées dans les trous 3212 des pieds de gerbage 321. La poignée 7 peut être réalisée en tout matériau approprié (matières plastiques, acier inoxydable, etc...).

Sur la figure 8a est représenté un moyen d'attache 6 sous forme d'une grenouillère réalisée dans un matériau présentant une élasticité et une résistance telles qu'elles assurent une bonne tenue du système de fermeture sur la nervure périphérique 311 du collier 31 et un dégagement aisé par simple déformation de ladite grenouillère.

Plus précisément la grenouillère 6 présente de l'arrière vers l'avant sur la vue 8a :
- un bord arrière roulé 61 recevant un axe d'articulation (non représenté) dont les bouts sont engagés dans les trous 3221 des supports d'articulation 322 précédemment décrits,
- une face supérieure 62 légèrement bombée munie d'un trou 63 pour le passage d'un oeillet de plombage solidaire du système de fermeture,
- un bord avant courbé 64,
- une patte avant 65, dirigée obliquement vers l'arrière,
- un bord courbé inversé 66 par rapport au bord courbé 64,
- et une lame de manoeuvre 67 s'étendant en hauteur sensiblement au-dessus du niveau de la face supérieure 62.

Par ces dispositions, on comprend qu'il suffit d'exercer avec les doigts une pression sur la lame de manoeuvre 67 pour déformer suffisamment la face supérieure 62 de la grenouillère et ainsi dégager le bord courbé inversé 66 de son appui élastique contre la nervure 311 du collier 31.

En définitive, le système d'attache préférentiel du mode de réalisation représenté sur les figures combine un couvercle basculant articulé avec deux attaches à grenouillères. L'axe d'articulation du couvercle est de préférence situé à la partie supérieure du tube, en l'espèce du carré de gerbage, pour faciliter l'extraction du linge.

L'association d'un couvercle basculant, articulé par le haut, avec deux attaches à grenouillères, constitue un système fiable, peu coûteux et ergonomique qui assure le respect de la limite d'asepsie et qui respecte les limites du gabarit de l'appareil.

Le système de fermeture 4 intégrant un dispositif de porte-filtre 5 est représenté sur les figures 3, 4a, 4b, 5a, 5b et 6.

Ce système se compose d'un couvercle 41, articulé dans les lumières oblongues 3211 des pieds de gerbage supérieurs 321 de manière à pouvoir basculer de bas en haut à l'ouverture et de haut en bas à la fermeture, un sur-couvercle 42 et des moyens de retenue du support de filtre 5 à l'intérieur du couvercle 41.

L'ensemble est conçu pour assurer une bonne circulation de la vapeur de stérilisation vers l'intérieur pour la stérilisation et une bonne extraction de la même vapeur pour le séchage, être une barrière microbienne de qualité, assurer l'inviolabilité de la fermeture depuis l'étape de stérilisation jusqu'à l'ouverture précédant l'utilisation du linge, permettre un nettoyage ou remplacement du filtre aisés, tout en rendant ce filtre inaccessible en condition fermée, coopérer avec les carrés de gerbage pour définir la valeur de la limite d'asepsie et la garantir, tout en étant simple et peu coûteux à fabriquer et en restant dans le gabarit limite de l'appareil, malgré sa position débordante par rapport au tube.

Le couvercle proprement dit 41 est délimité à sa base par une jupe circulaire 411 qui vient s'ajuster sur la nervure périphérique 311 du collier 31 portant les carrés de gerbage 3. Son plateau supérieur 412 comporte une vaste ouverture centrale délimitée 413 par un bourrelet 414 d'accrochage au sur-couvercle 42, et porte une nervure 415 concentrique au bourrelet 414. La paroi latérale 416 s'étend coniquement vers le haut entre la jupe 411 et le bord du plateau supérieur 412.

La partie supérieure de la jupe 411 porte une patte en équerre 417 dont le bord libre porte l'axe d'articulation 418 qui en service est tenu dans les lumières oblongues 3211 des pieds de gerbage 321.

Sur ses côtés verticaux, la jupe 411 comporte un entablement 419 sensiblement parallélépipédique qui fait fonction de butée d'accrochage pour la grenouillère 6. L'entablement 419 porte en saillie un oeillet de plombage 420 qui coopère avec le trou 63 de la grenouillère 6 pour la mise en place après fermeture d'un moyen d'inviolabilité tel qu'un fil plombé, une goupille arrêtée ou autre équivalent.

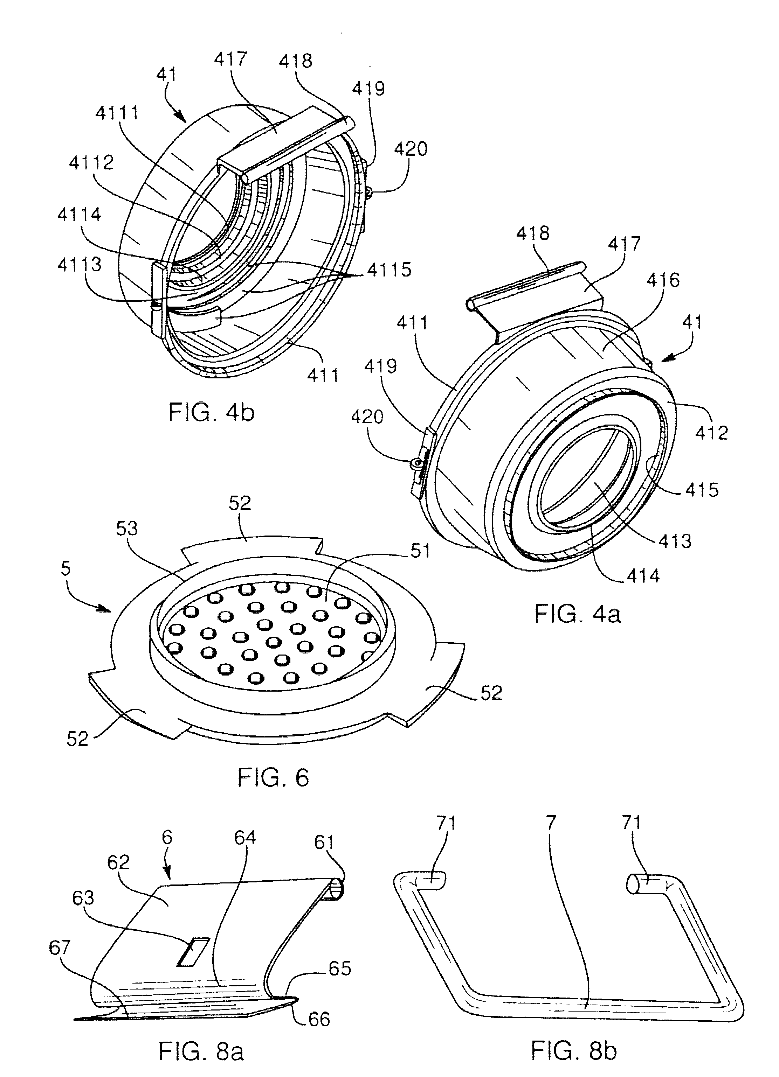

Vu de l'intérieur sur la figure 4b, le couvercle 41 présente depuis le trou 413 en direction de la jupe 411, une série de gradins à savoir : un premier gradin 4111 constituant un rebord d'accrochage au niveau du bourrelet 414, un deuxième gradin 4112 définissant avec un troisième gradin 4113 une rainure 4114 pour la mise en place d'un joint non représenté. Le troisième gradin 4113 fait fonction de piste pour des rampes 4115 destinées à coopérer avec des ailettes du support de filtre 5 pour bloquer par rotation ce dernier à l'intérieur du couvercle.

Vu de l'extérieur, sur la figure 5a, le sur-couvercle 42 est en forme d'auge tronconique présentant en son milieu un oeillet d'accrochage 421 pour une étiquette informative et dont le bord périphérique 422 recouvre le couvercle 41 en position de service sur au moins un quart de sa hauteur et en définissant avec lui une ouverture circulaire 423 pour le passage de la vapeur de stérilisation.

Vu de l'intérieur (figure 5b, le sur-couvercle 42 comporte deux pattes d'accrochage en saillie 424 dont le crochet terminal vient en service s'engager sous le gradin 4111 du couvercle 4, et quatre pieds en saillie 425 destinés à prendre appui sur le plateau supérieur 412 du couvercle 41 au niveau de la nervure 414. Par ces moyens, il est impossible d'avoir accès de l'extérieur aux moyens d'accrochage du sur-couvercle 42 sur le couvercle 41 et ainsi au filtre porté par le support 5. D'autre part, un couloir de circulation de vapeur est ménagé, qui autorise une bonne injection et extraction de la vapeur dans le tube 2 à travers le filtre. La nervure du 414 du couvercle 41, empêche l'introduction d'un instrument ou d'une tige quelconque dans l'ouverture 423 pour tenter de désolidariser de l'extérieur le couvercle et le sur-couvercle par appui sur les pattes d'accrochage 424. Les pieds d'appui 425 constituent également des obstacles qui rendent encore plus difficiles de telles tentatives d'effraction.

Le porte-filtre 5 représenté à la figure 6 est constitué par un disque perforé 51, muni à sa périphérie de 3 ailettes de fixation 52 biseautées en épaisseur et destinées à coopérer avec les rampes 4115 du couvercle pour le montage amovible du porte-filtre à l'intérieur dudit couvercle.

La face supérieure du disque 51 comporte une nervure circulaire en saillie 53 qui délimite un logement pour un filtre (non représenté), ainsi que la zone de perforations du disque 51.

En service le support de filtre 5 pourra être équipé de tout type de filtre convenable pour la stérilisation, agréé par la pharmacopée, et notamment des filtres en papier crêpé, des filtres en polytétrafluoéthylène (PTFE) ou des filtres en acier inox fritté.

La figure 3 représente en coupe verticale médiane l'ensemble du système de fermeture (couvercle 41, sur-couvercle 42 et support de filtre 5) en position de service, le filtre interchangeable n'étant pas représenté. On voit que la nervure 53 du support de filtre 5, coopère avec les gradins 4112 et 4113 du couvercle pour prendre appui sur le joint de la rainure 4114.

La description qui précède est donnée à titre d'exemple de réalisation, nullement limitatif, d'autres variantes de réalisation pouvant être imaginées sans sortir du cadre de l'invention.

## Revendications

1. Procédé de traitement de linge chirurgical soumis à stérilisation, dans lequel le paquet de linge à traiter est trié, décontaminé, lavé et séché avant d'être conditionné pour le passage en autoclave, caractérisé en ce que, avant stérilisation, ledit paquet de linge est roulé en cylindre et logé dans un tube comportant à au moins une de ses deux extrémités un système de fermeture, protégé contre les manipulations, muni d'un filtre, ledit tube étant équipé à sa périphérie de moyens de gerbage.

2. Appareil pour la mise en oeuvre du procédé selon la revendication 1, caractérisé en ce qu'il est constitué d'un corps tubulaire (2) muni à sa périphérie de moyens de gerbage (3) et comportant à au moins une de ses extrémités des moyens de fermeture (4), des moyens de support de filtre (5) pour un filtre interchangeable, portés par les moyens de fermeture, des moyens d'attache rapide (6) des moyens de fermeture (4) sur le tube (2), des moyens d'inviolabilité de la fermeture.

3. Appareil selon la revendication 2, caractérisé en ce que la longueur du tube est supérieure à celle du cylindre de linge roulé, de manière à constituer entre les moyens de fermeture 4 et ledit cylindre de linge un espace vide qui détermine la limite d'asepsie.

4. Appareil selon la revendication 1 ou 2, caractérisé en ce que les moyens de fermeture (4) sont constitués par un couvercle (41) et un sur-couvercle (42) assemblés entre eux par des moyens qui à la fois rendent toute tentative de désolidarisation du couvercle (41) et du sur-couvercle (42), de l'extérieur sans outils spéciaux, impossible, et maintiennent un couloir suffisant entre le couvercle et le sur-couvercle pour une bonne circulation des flux de vapeur lors de la stérilisation.

5. Appareil selon la revendication 4, caractérisé en ce que le sur-couvercle (42) s'étend autour du couvercle (41) sur au moins un quart de la hauteur de ce dernier.

6. Appareil selon l'une quelconque des revendications 2 à 5, caractérisé en ce que les moyens de fermeture (4) comportent des moyens de fixation amovibles (4115, 52) du support de filtre.

7. Appareil selon l'une quelconque des revendications 2 à 6, caractérisé en ce que les moyens de fermeture (4) sont articulés par rapport au tube (2) de manière à pouvoir basculer de bas en haut à l'ouverture et sont combinés avec des moyens d'attache rapide portés par le tube (2).

8. Appareil selon la revendication 7, caractérisé en ce que les moyens d'attache rapide sont des moyens à grenouillère déformable (6).

9. Appareil selon l'une quelconque des revendications 2 à 8, caractérisé en ce que les moyens de gerbage (3), les moyens de fermeture (4), et les moyens d'attache (6) sont portés par un collier (31) fixé à l'extérieur du tube (2) à proximité des extrémités dudit tube (2).

10. Appareil selon l'une quelconque des revendications 2 à 9, caractérisé en ce qu'il est équipé d'un filtre en acier inoxydable fritté.
